# EUROPÄISCHE PATENTSCHRIFT

(11) **EP 0 744 919 B1**
(45) Veröffentlichungstag und Bekanntmachung des Hinweises auf die Patenterteilung: **06.05.1998**
(21) Anmeldenummer: 95910701.2
(22) Anmeldetag: 15.02.1995
(51) Int. Cl.: A61B 17/17

(54) **VORRICHTUNG ZUR ARTHROSKOPISCHEN WIEDERANHEFTUNG VON GERISSENEN GEWEBEN, INSBESONDERE VON GERISSENEN ROTATORENMANSCHETTEN**
DEVICE FOR THE ARTHROSCOPIC REATTACHMENT OF TORN TISSUES, IN PARTICULAR OF TORN ROTATOR SHEATHS
DISPOSITIF DE REFIXATION ARTHROSCOPIQUE DE TISSUS DECHIRES, EN PARTICULIER DE GAINES DE ROTATEURS DECHIREES

(30) Priorität: 17.02.1994 US 197829; 26.08.1994 US 288228
(43) Veröffentlichungstag der Anmeldung: 04.12.1996
(73) Patentinhaber: ARTHREX, INC., Naples, FL 33942 (US); Habermeyer, Peter, Dr., 69120 Heidelberg (DE)
(72) Erfinder: HABERMEYER, Peter, D-69120 Heidelberg (DE); SCHMIEDING, Reinhold, Naples, FL 33963 (US); KRUPP, Stefan, D-80999 München (DE)
(74) Vertreter: Richter, Joachim, Dipl.-Ing.
(86) Internationale Anmeldenummer: IB9500182
(87) Internationale Veröffentlichungsnummer: WO9522288

(56) Entgegenhaltungen:
- US-A- 5 250 055
- US-A- 5 269 786

## Beschreibung

Die Erfindung betrifft eine Vorrichtung zur arthroskopischen Wiederanheftung von gerissenem Gewebe, insbesondere von gerissenen Rotatorenmanschetten,an den Knochen an einer chirurgisch zu operierenden Stelle mit einem mit einem Faden versehenen Nahtanker, einem Gewebegreifer an einer mit einem Griff versehenen Hülse und einer an dem Gewebegreifer vorgesehenen Einrichtung zum Zurückziehen des Fadens.

Die Rotatorenmanschette (Sehnen der Muskelkappe) wird von den Mm. supraspinatus, infraspinatus, teres minor und subscapularis gebildet, deren Sehnen miteinander und gegen die Schultergelenkkapsel fusionieren und eine muskulotendinöse Kapsel bilden, welche an beiden Tubercula im kranialen Anteil des anatomischen Humerushalses inseriert, zwischen denen die lange Bizepssehne unter der Rotatorenmanschette hindurchtritt. Schmerzen und Bewegungsstörungen der Schulter finden oftmals ihre Ursache insbesondere in Erkrankungen der Supraspinatussehne, die unter dem Akromion über den Oberarmkopf hinweg zum Tuberculum majus humeri zieht. Zwischen ihr und dem Akromion liegt die Busa subacromialis. Bei einem vollen Riß der Rotatorenmanschette ist die vollständige aktive Abduktion des Armes eingeschränkt.

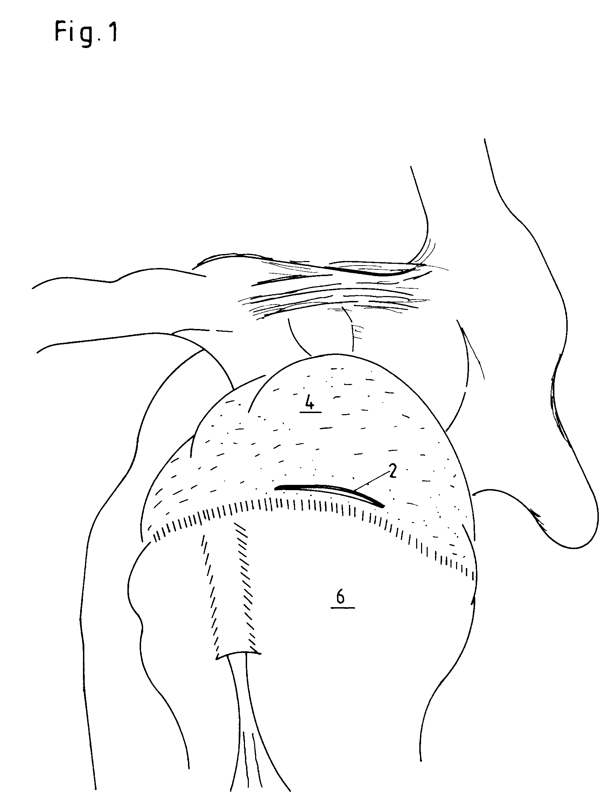

Wie in Fig. 1 gezeigt, ist es beim Auftreten eines Risses 2 in der Rotatorenmanschette 4 der Schulter notwendig, die gerissene Sehne wieder an den Knochen des Humeruskopfes 6 anzuheften.

Es sind verschiedene Wiederherstellungsverfahren bekannt. Die bekannten Wiederanheftungsprozeduren sind jedoch langwierig und kompliziert. In einem chirurgischen Verfahren, das von Stephen J. Snyder, MD, vom Southern California Orthopedic Institute in der Veröffentlichung "The Revo Rotator Cuff Fixation System", Linvatec Corp. (1993), beschrieben wurde, werden zunächst bereits mit Faden versehene Schrauben in den Knochen gebohrt. Der durch jede Schraube geführte Faden wird dann unter Benutzung eines Fadenfängers von der zu operierenden Stelle zurückgezogen. Eine Nahtstanze wird zum Punktieren der gerissenen Manschette benutzt. Ein Pendelmechanismus wird durch die Manschette geführt und unter Benutzung eines Greifers aus der Vorderkanüle zurückgezogen. Eine Öse im Pendelmechanismus wird mit einem freien Ende des durch eine der Schrauben verlaufenden Fadens versehen. Der Pendelmechanismus wird durch die Manschette und unter Benutzung des Greifers aus der Vorderkanüle gezogen. Diese Prozedur wird mit dem zweiten freien Ende des durch die Schraube verlaufenden Fadens wiederholt. Danach wird das Paar Nahtstränge aus der Seitenkanüle zurückgezogen. Die oben beschriebene Prozedur wird solange wiederholt, bis alle Nahtstränge aus den Schrauben durch den Riß geführt sind. Die Nahtstränge werden dann verknotet und zusammengebunden, um die wiederangeheftete Rotatorenmanschette zu sichern.

Dieses chirurgische Verfahren ist ein "offenes" Verfahren. Wie bei den meisten offenen Verfahren kann eine Morbidität der zu operierenden Stelle auftreten. Ein weiterer Nachteil dieses Verfahrens besteht darin, daß getrennte Greif- und Punktierungsinstrumente benötigt werden. Hinzu kommt, daß bei diesem Verfahren beim Nähen das Gewebe bekanntermaßen unter Benutzung von Nadelhaltern ergriffen wird (US-A-5,304,203). Die Nadelhalter nach US-A-2,665,692 schließen auch Backen 13 und 14 ein, durch deren Öffnungen der Chirurg eine Nadel führen kann. Die Nadelhalter dieses Patents sind jedoch nicht für arthroskopische chirurgische Prozeduren geeignet.

Durch die US-A-5,250,055 ist für die arthroskopische Chirurgie eine Vorrichtung zum Anheften einer Sehne an einen Knochen durch das darüberliegende Bindegewebe bekannt. Diese Vorrichtung besteht aus einer Kanüle mit einer griffartigen Handhabe, um einen Durchgang für das Einführen der Heftungsvorrichtung zu schaffen, und die in das Gewebe eingeführt wird, wobei sie in den Knochen eindringt, wo ein Faden angeheftet werden soll. Zum Einführen in das Gewebe ist diese Kanüle mit einem abnehmbaren Stopfen versehen. In die Kanüle ist ein Führungselement einführbar, das zwei im Abstand über einen Steg gehalten, parallel zueinander verlaufende Hülsen mit Längsdurchbohrungen aufweist. Für den Anheftungsvorgang wird ein Fadenhalter in eine der beiden Längsdurchbohrungen des Führungselementes eingeführt. Dieser Fadenhalter weist einendseitig einen kurzen abgewinkelten Abschnitt mit einer Öse zur Aufnahme des Fadens auf. Das andere freie Ende des Fadenhalters ist entgegengesetzt zum Abschnitt mit der Öse als Handhabe abgewinkelt. Für den Anheftvorgang wird die Kanüle in das Gewebe eingeführt und zwar bis an den Knochen, wo der Faden angeheftet werden soll. Im Anheftungsbereich wird die anzuheftende Sehne von der Kanüle ergriffen und gehalten. Daraufhin wird das Führungselement in die Kanüle eingeführt. Mittels eines erst in die eine Längsdurchbohrung und dann in die andere Längsdurchbohrung des Führungselementes eingeführten Bohrers werden zwei Bohrungen in Verlängerung der Längsdruchbohrungen am Knochen angebracht. Nach dem Einführung eines Fadens in die Öse des Fadenhalters wird dieser in eine der beiden Längsdurchbohrungen eingeführt. Das den Faden tragende Ende des Fadenhalters besteht aus federndelastischem Material, so daß der beim Einführen in die Längsdurchbohrung gestreckte sonst abgewinkelte Fadenhalterabschnitt mit der Öse nach dem Austritt am Ende der Längsdurchbohrung innerhalb der Knochenbohrung wieder in seine vorgegebene abgewinkelte Stellung zurückfedert und in die zweite Knochenbohrung eindringt. Sobald die die Öse tragende Spitze des Fadenhalters in die zweite Knochenbohrung eindringt, wird ein Ende des Fadens mittels eines Fadenfängers durch die freie Längsdurchbohrung des Führungselementes zurückgezogen. Danach wird der Fadenfänger mit dem Führungselement und dem fadenhalter einschließlich der Kanüle zurückgezogen; die freien Enden des Fadens werden miteinander verknotet. Diese Vorrichtung zur arthroskopischen Anheftung von gerissenem Gewebe oder Sehnen an einem Knochen umfaßt mehrere Einzeleinrichtungen, die entsprechend dem Fortgang des Anheftungsvorganges vom Operateur eingesetzt werden, so daß zahlreiche Handgriffe erforderlich sind, wodurch keine Zeiteinsparung gegeben ist.

Alle vorangehend erörterten Verfahren und Vorrichtungen nach dem Stand der Technik erfordern getrennte Instrumente zum Ergreifen des wiederherzustellenden Gewebes und zum Zurückziehen des Fadens von der arthroskopisch zu operierenden Stelle. So ist es bisher notwendig gewesen, zahlreiche unterschiedliche chirurgische Vorrichtungen auf sehr engem Raum zu benutzen.

Die vorliegende Erfindung hat zur Aufgabe, eine Vorrichtung zur arthroskopischen Wiederanheftung von gerissenem Gewebe zu schaffen, wobei die oben erwähnten Mängel des Verfahrens nach dem Stand der Technik vermieden werden.

Gelöst wird diese Aufgabe bei einer Vorrichtung gemäß der eingangs beschriebenen Art mit den im Kennzeichen des Anspruches 1 angegebenen Merkmalen.

Mit dieser Vorrichtung wird gerissenes Gewebe unter Benutzung von mindestens einem Nahtanker und einem Gewebegreifer wieder an den Knochen angeheftet.Der Gewebegreifer ergreift das gerissene Gewebe mit Backen. Ein erster mit Faden versehener Nahtanker wird mit einer einzigen Bohrung durch die Backen des Greifers, das ergriffene Gewebe und in den Knochen eingeführt. Wenn der Greifer von der zu operierenden Stelle entfernt wird, ziehen die Backen des Greifers gleichzeitig den Faden von der zu operierenden Stelle zurück.

Der Gewebegreifer schließt eine verlängerte Hülse mit zwei gegenüberliegenden Enden ein, wobei das erste Ende an der zu operierenden Stelle in einen Patienten einführbar ist. Zwei drehbare Backen zum Ergreifen des gerissenen Gewebes befinden sich am ersten Ende der Hülse. Die Backen schließen eine obere und eine untere Backe ein. Die obere Backe hat eine Öffnung, und die untere Backe schließt eine U-förmige Nut ein. Die Öffnung und die Nut werden aufeinander ausgerichtet, wenn die Backen geschlossen werden.

Ein ausziehbarer Stützfuß zum Führen des Bohrers, der sich an der Hülse befindet, dient der Aufnahme und der Ausrichtung des mit Faden versehenen Nahtankers, so daß der Nahtanker automatisch auf die Öffnung und die Nut der Backen des Greifers an der zu operierenden Stelle ausgerichtet ist.

Ein besonderer Vorteil der Vorrichtung besteht darin, daß der Chirurg mit Hilfe des Greifers mit einer einzigen Bohrung das Gewebe ergreifen, den Faden durch das ergriffene Gewebe einführen und den Nahtanker in den Knochen bohren kann. Somit ist es erfindungsgemäß nicht notwendig, daß der Chirurg eine Nadel mit Faden versieht, die mit Faden versehene Nadel erneut in die zu operierende Stelle einführt und das gerissene Gewebe näht. Damit wird die Operationszeit wesentlich verkürzt.

Ein weiterer Vorteil besteht darin, daß der Greifer automatisch den Faden von der zu operierenden Stelle zurückzieht, wenn der Greifer selbst entfernt wird. Deshalb ist ein getrenntes Instrument für das Zurückziehen des Fadens nicht erforderlich.

Weiterbildungen der Erfindung sind in den Unteransprüchen beschrieben.

Ausführungsbeispiele der Erfindung sind in den Zeichnungen dargestellt. Es zeigen
- Fig. 1: eine gerissene Rotatorenmanschette,
- Fig. 2: eine perspektivische Ansicht des erfindungsgemäßen Greifers, die die von den Backen des Greifers gehaltene Rotatorenmanschette zeigt,
- Fig. 3a bis 3c: in verschiedenen Abschnitten die Anwendung des Gerätes bei der Durchführung des chirurgischen Verfahrens,
- Fig. 4: einen Längsschnitt des Greifers,
- Fig. 5: einen vergrößerten Teillängsschnitt der Backen des Greifers,
- Fig. 6: eine entlang der Linie A-A in Fig. 5 vorgenommene Unteransicht der Backen,
- Fig. 7: eine entlang der Linie B-B in Fig. 5 vorgenommene Draufsicht der Backen,
- Fig. 8: einen Seitenteilquerschnitt des Stützfußes,
- Fig. 9: eine entlang der Linie C-C in Fig. 8 vorgenommene Vorderansicht des Stützfußes,
- Fig. 10: einen Teilquerschnitt der durch den Stützfuß aufgenommenen Kanüle,
- Fig. 11: eine Einfädelvorrichtung für die Vorrichtung und das Verfahren der vorliegenden Erfindung,
- Fig. 12: einen Vorrichtungstreiber für die Vorrichtung und das Verfahren der vorliegenden Erfindung und
- Fig. 13: einen Nahtanker für die Vorrichtung und das Verfahren der vorliegenden Erfindung.

Die vorliegende Erfindung bezieht sich auf eine Vorrichtung für die arthroskopische Wiederanheftung und Wiederherstellung von gerissenem Gewebe und Knochen an einer chirurgisch zu operierenden Stelle unter Benutzung eines Gewebegreifers.

Obwohl die vorliegende Erfindung in erster Linie im Zusammenhang mit der Wiederherstellung einer gerissenen Rotatorenmanschette beschrieben wird, sind Vorrichtung und Verfahren auch für die arthroskopische Bankart-Schulterwiederherstellung sowie für die Ellenbogen-, Knie-, Knöchel- und Hüftchirurgie und andere chirurgische Verfahren einsetzbar.

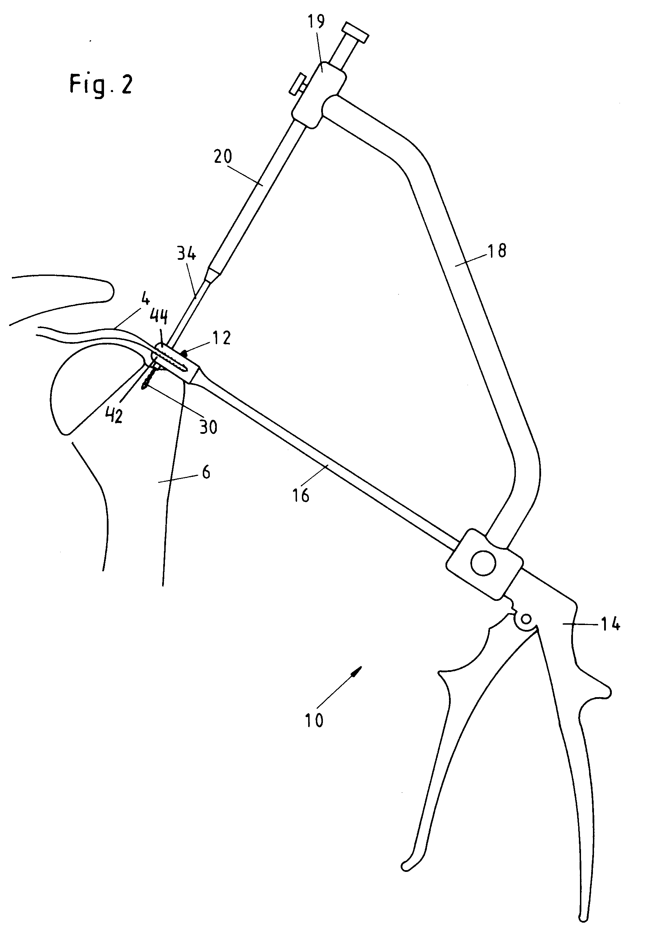

Wie Fig. 2 zeigt, ist ein Greifer 10 mit schwenkbaren Backen 12 zum Ergreifen einer gerissenen Rotatorenmanschette 4 versehen. Die Backen 12 befinden sich am distalen Ende einer hohlen Hülse 16. Ein Greifstück 14 befindet sich am proximalen Ende der Hülse 16. Ein ausziehbarer Stützfuß zur Führung des Bohrers 18 reicht bis über die Hülse 16 hinaus und wird von ihr gestützt.

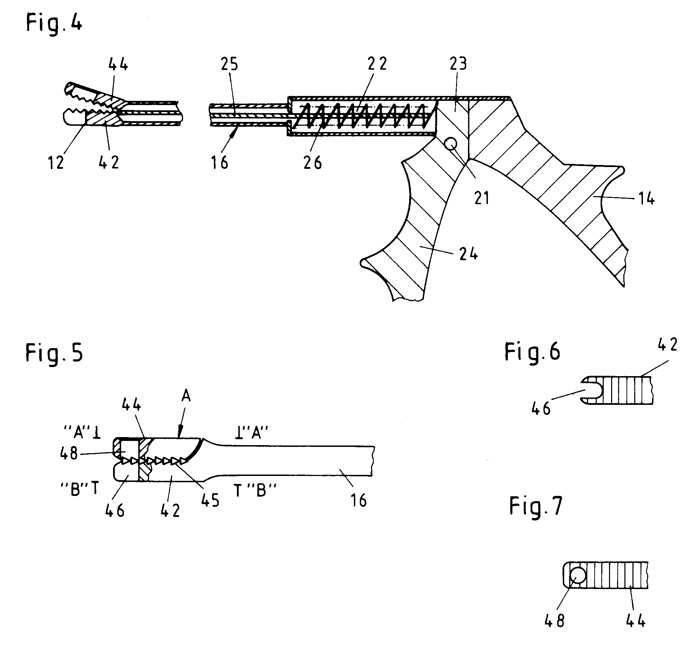

Bezugnehmend auf Fig. 5 bis 7 schließen die sich am distalen Ende der Hülse 16 befindenden Backen 12 eine untere, feststehende Backe 42 und eine obere, bewegliche Backe 44 ein. Beide Backen 42 und 44 schließen Zähne 45 zum Erfassen des Gewebes ein.

Nach Fig. 6 ist die untere, feststehende Backe 42 mit einer U-förmigen Nut 46 und die bewegliche Backe 44 mit einer vollständig eingefaßten Öffnung 48 versehen (Fig. 7). Wenn sich die Backen, wie in Fig. 5 gezeigt, in geschlossener Position befinden, sind die Nut 46 und die Öffnung 48 aufeinander ausgerichtet, damit der Nahtanker hindurchgeführt werden kann. Wie im folgenden ausgeführt, wird mindestens ein, bevorzugterweise resorbierbarer, durch die Backen 12 geführter Faden in der Öffnung 48 festgehalten und gleichzeitig von der zu operierenden Stelle entfernt, nachdem der Anker eingebettet worden ist.

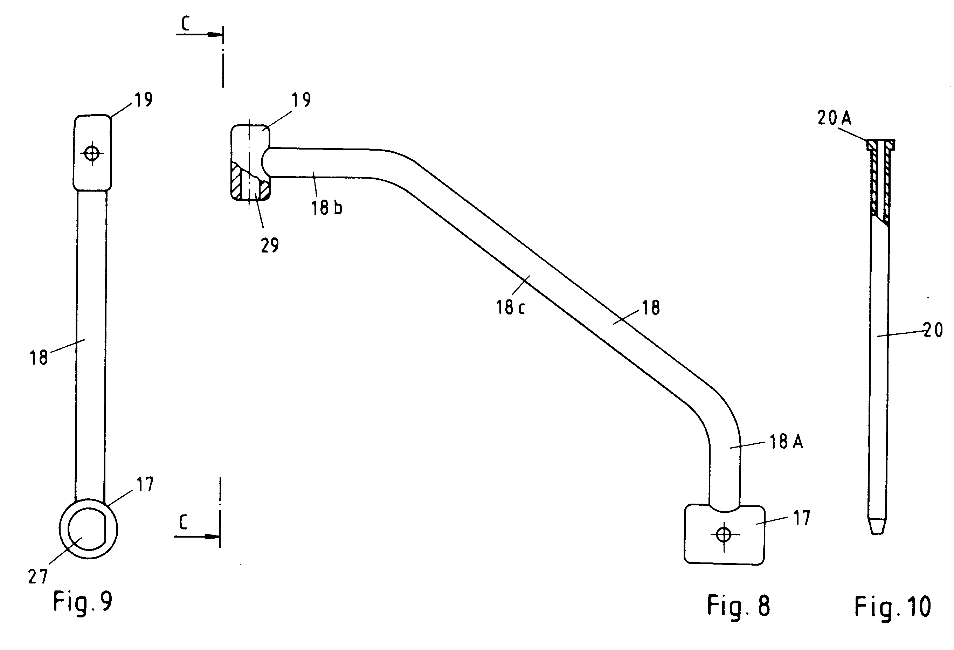

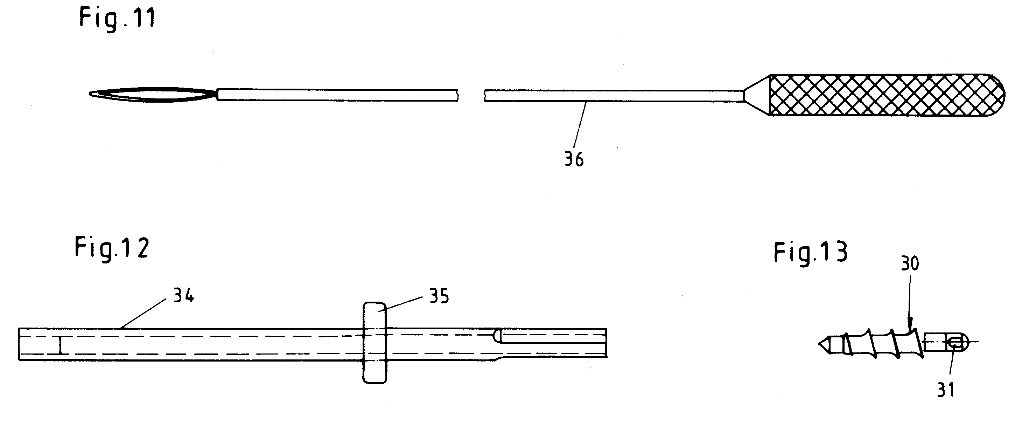

Entsprechend Fig. 8 bis 10 hat der ausziehbare Stützfuß 18 ein unteres Ende 17 und ein oberes Ende 19. Das untere Ende 17 schließt einen hohlen Abschnitt 27 ein, durch den sich die Hülse 16 erstreckt. Das obere Ende 19 ist mit einer Kanüleneinführung 29 zur Aufnahme der Kanüle 20 versehen. Die Kanüle 20 wiederum nimmt einen Vorrichtungstreiber 34 auf (Fig. 2, 12), der mit einem mit Faden versehenen Nahtanker 30 bestückt ist, der in den Knochen 6 eingebettet werden soll. Der Nahtanker 30, im Detail gezeigt in Fig. 13, ist eine selbstschneidende Schraube. Deshalb ist kein Vorbohren von Führungslöchern in den Knochen notwendig. Der ausziehbare Stützfuß 18 hat einen ersten Schenkel 18A, der sich senkrecht vom unteren Ende 17 erstreckt, einen zweiten Schenkel 18B, der sich waagerecht vom oberen Ende 19 erstreckt, und einen Verbindungsschenkel 18C, der sich zwischen den Schenkeln 18A und 18B erstreckt. Wie im folgenden ausgeführt, ist der ausziehare Stützfuß 18 so gestaltet, daß dieser automatisch die Kanüle 20 und somit die durch diese verlaufenden Treiber 34 und Nahtanker 30 auf die Öffnung 48 und die Nut 46 in den Backen ausrichtet.

Der Vorrichtungstreiber 34 ist mit einem einstellbaren Tiefenanschlag 35 versehen (Fig. 12), mit dessen Hilfe der Chirurg die Tiefe der Implantation des Nahtankers 30 steuern kann. Wie in Fig. 10 gezeigt, hat die Kanüle 20 an ihrem proximalen Ende eine Lippe 20A zur Aufnahme des Treibers 34. Der Tiefenanschlag 35 verhindert mit Hilfe der Lippe 20A das weitere Vordringen des Ankers.

Beim operativen Einsatz wird der Greifer 10 in einen Eingang in der Schulter eingeführt. Der Eingang wird geöffnet, indem zunächst ein Schnitt in die Haut gemacht und dann eine Kanüle (nicht dargestellt) durch den Schnitt eingeführt wird. Das distale Ende der hohlen Hülse 16 wird durch die Kanüle eingeführt, bis die Backen 12 das gerissene Rotatorenmanschettengewebe erreichen. Nachdem das Instrument in Position gebracht worden ist, wird der Greifer 10 so mit Hilfe des Griffstückes 14 bedient, daß das gerissene Gewebe 4 von den Backen 12 ergriffen wird.

Danach wird die Kanüle 20 nach unten geschoben, bis sich die Spitze, wie in Fig. 3A gezeigt, auf einer Ebene mit der Haut des Patienten befindet. Wie bereits erwähnt, ist der ausziehbare Stützfuß 18 so gestaltet, daß die Kanüle 20 automatisch auf die Nut 46 und die entsprechende Öffnung 48 in der unteren Backe 42 bzw. oberen Backe 44 des Greifers ausgerichtet wird.

Anschließend wird ein Nahtanker 30 mit dem Faden der richtigen Größe versehen, welcher durch die Öse 31 geführt wird. Eine Einfädelvorrichtung 36 ist, wie in Fig. 11 gezeigt, zur Bewerkstelligung des Einfädelns des Fadens durch den Vorrichtungstreiber 34 verwendbar.

Das Ösenende des nun mit dem Faden 8 versehenen Nahtankers wird dann in den Vorrichtungstreiber 34 eingesetzt. Der Vorrichtungstreiber 34, an dessen distalem Ende sich der Nahtanker 30 befindet, wird durch die Kanüle 20 in einen Schnitt im Patienten eingeführt. Ein motorisch angetriebener Bohrer wird am Treiber 34 zum Drehen des Treibers angeschlossen und der Nahtanker 30 am distalen Ende des Treibers befestigt. Der Nahtanker 30 wird durch die Öffnung 48 in der oberen Backe 44 und die Nut 46 in der unteren Backe 42 geschoben und mit einer einzigen Bohrung durch das ergriffene Gewebe und in den Knochen gebohrt (Fig. 2).

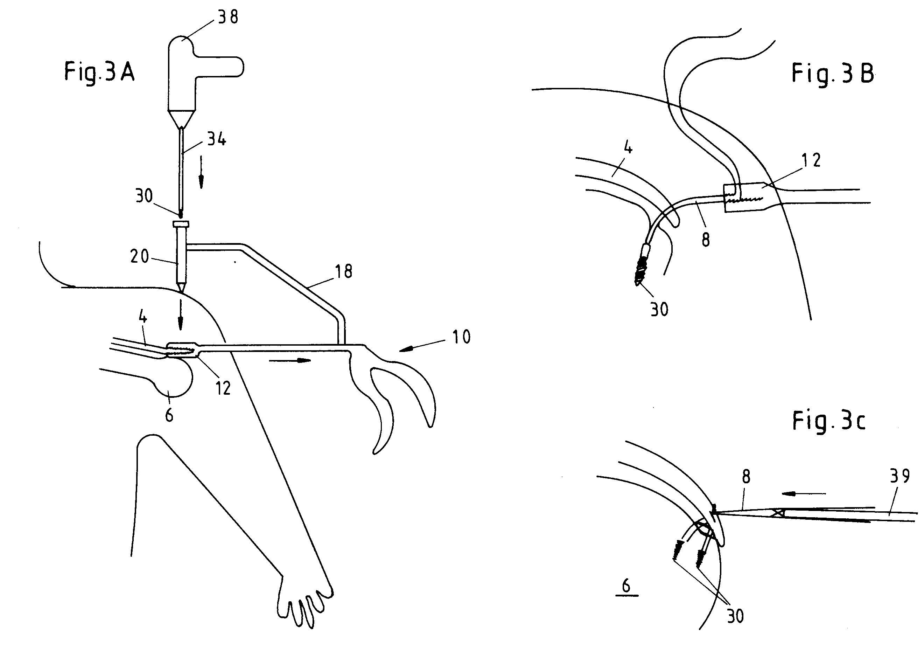

Wenn der Nahtanker 30 in den Knochen gebohrt ist, wird der Treiber 34 entfernt. Bezugnehmend auf Fig. 3B wird der Greifer 10 von der zu operierenden Stelle entfernt und der mit Faden versehene Nahtanker in seiner Position gelassen. Wenn der Greifer 10 zurückgezogen wird, wird gleichzeitig der durch die Öffnung 48 in der oberen Backe 44 verlaufende Faden 8 von der zu operierenden Stelle entfernt.

Für gewöhnlich wird die ganze Prozedur wiederholt, um weitere mit Faden versehene Nahtanker einzuführen. Wie in Fig. 3B und 3C gezeigt, erstreckt sich die dopelsträngige Naht 8 von den Ankern 30 durch das gerissene Rotatorenmanschettengewebe 4. Es werden Matratzennähte gemacht, indem ein Strang der Naht von einem der Anker mit dem anderen Anker und umgekehrt verbunden werden. Die Knoten können verbunden und unter Benutzung eines Knotendrückers 39 in die Schulter geschoben werden, wie in US-A-5,176,691 beschrieben.

Der Greifer 10 ist wie folgt aufgebaut:

Das Griffstück 14 des Greifers 10 schließt einen Auslöser 24 ein, der durch einen Drehbolzen 21 drehbar mit dem Griffstück verbunden ist. Entsprechend Fig. 4 schließt der Auslöser 24 eine Verlängerung 23 ein, die sich in den hohlen Abschnitt 22 des Griffstücks bewegt. Die Hülse 16 wird an einem Ende am Griffstück befestigt. Bei der Benutzung wird das distale Ende des Greifers an der zu operierenden Stelle gegen das zu ergreifende Gewebe gerichtet. Die bewegliche Backe 44 wird durch Zusammendrücken des Auslösers 24 in Richtung der feststehenden Backe 42 bewegt. Wenn sich der Auslöser 24 durch das Drehen um den Drehbolzen 21 nach innen bwegt, wird die Verlängerung 23 gegen den Stab 25 gedrückt, wodurch der Stab 25 nach vorn in Richtung des distalen Endes der Hülse 16 gegen die Kraft der Feder 26 geschoben wird. Wenn der Stab 25 nach von gedrückt wird, dreht sich die bewegliche Backe 44 in Richtung der feststehenden Backe 42, um die Backen zu schließen. Wenn der entsprechende Gewebeabschnitt isoliert und von den Backen 42 und 44 ergriffen ist, kann der Auslöser 24 in seiner geschlossenen Position mit einem nicht dargestellten Arretiermechanismus verriegelt werden.

## Patentansprüche

1. Vorrichtung zur arthroskopischen Wiederanheftung von gerissenem Gewebe, insbesondere von gerissenen Rotatorenmanschetten, an den Knochen an einer chirurgisch zu operierenden Stelle mit einem mit einem Faden (8) versehenen Nahtanker (30), einem Gewebegreifer (10) an einer mit einem Griff (14) versehenen Hülse (16) und einer an dem Gewebegreifer vorgesehenen Einrichtung zum Zurückziehen des Fadens,
dadurch gekennzeichnet,
daß die Hülse (16) an ihrem proximalen Ende den Griff (14) und an ihrem distalen, in den Patienten an der zu operierenden Stelle einführbaren Ende schwenkbare Backen (12) zum Ergreifen des gerissenen Fadens trägt, daß an dem Gewebegreifer (10) die Einrichtung zum Zurückziehen des mit dem Faden (8) versehenen Nahtankers (30) und des an dem mit dem Faden versehenen Nahtankers (30) befindlichen Fadens (8) von der Operationsstelle bei Entfernung des distalen Endes der Hülse (16) von der Operationsstelle angeordnet ist, wobei der mit dem Faden (8) versehene Nahtanker (30) mit einer einzigen Bohrung bei gleichzeitigem Erfassen des Gewebes mittels des Gewebegreifers (10) in den Knochen eingeführt wird, daß an dem distalen Ende des Gewebegreifers (10) zwei verschwenkbare Backen (12) zum Ergreifen des gerissenen Gewebes vorgesehen sind, wobei die Backen (12) von einer oberen Backe (44) und einer unteren Backe (42) gebildet werden, von denen die obere Backe (44) mit einer Öffnung (48) und die untere Backe (42) mit einer U-förmigen Nut (46) versehen sind, wobei die Öffnung (48) und die Nut (46) aufeinander ausgerichtet werden, wenn die Backen (42,44) geschlossen und wobei der mit dem Faden (8) versehene Nahtanker (30) durch die aufeinander ausgerichteten Öffnung (48) und Nut (46) der Backen (42,44) mit erfaßtem, gerissenem Gewebe in den Kochen eingeführt wird.

2. Vorrichtung nach Anspruch 1,
dadurch gekennzeichnet,
daß der mit dem Faden (8) versehene Nahtanker (30) durch die aufeinander ausgerichteten Öffnung (48) und Nut (46) der Backen (42,44) unter Verwendung einer an der Vorrichtung vorgesehenen Kanüle (20) zur Führung des Nahtankers (30) mit erfaßtem, gerissenem Gewebe in den Knochen eingeführt wird, wobei die Kanüle (20) an dem freien Ende eines mit dem proximalen Ende der Hülse (16) verbundenen ausziehbaren Stützfußes (18) befestigt ist.

3. Vorrichtung nach Anspruch 2,
dadurch gekennzeichnet,
daß der ausziehbare Stützfuß (18) zur Aufnahme eines Bohrers dient, so daß der mit dem Faden (8) versehene Nahtanker (30) automatisch auf die Öffnung (48) und die Nut (46) der Backen (42, 44) des Greifmittels an der zu operierenden Stelle ausrichtbar ist.

4. Vorrichtung nach einem der Ansprüche 2 und 3,
dadurch gekennzeichnet,
daß der ausziehbare Stützfuß (18) ein unteres Ende (17) und ein oberes Ende (19) aufweist, wobei das untere Ende (17) hohl ist, um die Hülse (16) des Greifers (10) aufzunehmen, wobei das obere Ende (19) mit einer Kanüleneinführung (29) zur Aufnahme einer Kanüle (20) versehen ist, durch die der mit dem Faden (8) versehene Nahtanker (30) in Richtung der zu operierenden Stelle geschoben wird.

5. Vorrichtung nach einem der Ansprüche 2 bis 4,
dadurch gekennzeichnet,
daß der ausziehbare Stützfuß (18) einen ersten Schenkel (18A), der sich vom unteren Ende (17) erstreckt, einen zweiten Schenkel (18B), der sich vom oberen Ende (19) erstreckt, und einen Verbindungsschenkel (18C), der den ersten Schenkel (18A) mit dem zweiten Schenkel (18B) verbindet, aufweist.

6. Vorrichtung nach einem der Ansprüche 1 bis 5,
dadurch gekennzeichnet,
daß die Vorrichtung ein hohles Griffstück (14) aufweist, das ein vorderes Ende und ein rückwärtiges Ende aufweist, die einander gegenüberliegen, wobei das proximale Ende der verlängerten Hülse (16) am vorderen Ende des Griffstücks (14) befestigt ist und daß die untere Backe (42) an der Hülse (16) am distalen Ende befestigt ist, wobei ein verlängerter Stab (25) beweglich innerhalb der Hülse (16) gelagert ist, wobei der Stab (25) am distalen Ende an der oberen Backe (44) befestigt ist.

7. Vorrichtung nach einem der Ansprüche 1 bis 6,
dadurch gekennzeichnet,
daß das Griffstück (14) des Greifers (10) einen Griff und einen drehbar verbundenen, federbelasteten Auslöser (24) aufweist.

8. Vorrichtung nach Anspruch 7,
dadurch gekennzeichnet,
daß sich eine Verlängerung (23) des Auslösers (24) in das hohle Griffstück (14) des Greifers (10) erstreckt und mit dem Stab (25) zusammenwirkt, um den Stab (25) nach vorn in die Hülse (16) zu bewegen, wenn der Auslöser (24) gezogen bzw. bewegt wird.

9. Vorrichtung nach Anspruch 8,
dadurch gekennzeichnet,
daß die Vorrichtung eine Feder (26) aufweist, die sich auf dem Stab (25) zwischen der Verlängerung (23) des Auslösers (24) und der Hülse (16) befindet, daß, wenn der Auslöser (24) gezogen bzw. bewegt wird, sich die Verlängerung (23) innerhalb des Griffstücks (24) entgegen dem Federdruck nach vorn dreht, wobei sie den Stab (25) nach vorn bewegt, so daß sich die obere Backe (44) in Richtung zu der unteren Backe (42) der Hülse (16) bewegt, um das Gewebe zu ergreifen, und daß, wenn der Auslöser (24) losgelassen wird, die Verlängerung (23) und der Stab (25) zurückgedrückt und der Auslöser (24) und die bewegliche obere Backe (44) in eine normal offene Position gedrückt werden.

## Claims

1. Device for the arthroscopic reattachment of torn tissue, more particularly of torn rotator cuffs, to the bone at a point to be surgically operated with a suture anchoring means (30) provided with a suture (8), a tissue seizing means (10) on a sleeve (16) fitted with a gripping handle (14) and a means provided on the tissue seizing means for withdrawing the suture,
characterized in that
the sleeve (16), at its proximal end, carries the handle (14) and, at its distal end to be introduced into the patient at the point to be operated, swivellable jaws (12) for seizing the torn tissue, in that, on the tissue seizing means (10), the device for the withdrawal of the suture anchoring means (30) provided with the suture (8) located on the suture ancoring means (30) from the operating point is disposed, in which case the suture anchoring means (30) provided with the suture (8) is introduced into the bone with a single bore hole while simultaneously seizing the tissue with the aid of the tissue seizing means (10), in that, at the distal end of the tissue seizing means (10), two swivellable jaws (12) are provided for seizing the torn tissue, in which case the jaws (12) are constituted of an upper jaw (44) and a bottom jaw (42, of which the upper jaw (44) is provided with an aperture (48) and the bottom jaw (42) with a U-shaped groove (46), the aperture (48) and the groove (46) being aligned with each other when the jaws (42,44) are closed and whereby the suture anchoring means (30) provided with the suture (8) is introduced into the bone with the seized, torn tissue through the mutually aligned aperture (48) and groove (46) of the jaws (42,44).

2. Device according to Claim 1,
characterized in that
the suture anchoring means (30) provided with the suture (8) is introduced through the reciprocally aligned aperture (48) and groove (46) of the jaws (42,44) while use is made of a cannula (20) provided on the device for guiding the suture anchoring means (30) with the sized, torn tissue into the bone, while the cannula (20) is attached to the free end of a telescoping supporting foot or base (18) connected to the proximal end of the sleeve (16).

3. Device according to Claim 2,
characterized in that
the telescoping supporting foot or base (18) serves for accommodating a drill so that the suture anchoring means (30) provided with the suture (8) can be automatically aligned with the aperture (48) and the groove (46) of the jaws (42,44) of the seizing means at the point to be operated.

4. Device according to Claims 2 and 3,
characterized in that
the telescoping foot or base (18) possesses a lower end (17) and an upper end (19), with the lower end (17) being hollow in order to receive the sleeve (16) of the seizing means (10), white the upper end (19) is provided with a cannula inserting means (29) for receiving a cannula (20), through which the suture anchoring means (30) with the suture is thrust in the direction of the point to be operated.

5. Device according to any of Claims 2 to 4,
characterized in that
the telescoping supporting foot or base (18) possesses a first leg (18A) which extends from the lower end (17), a second leg (18B) which extends from the upper end (19) and a connecting leg (18C), which connects the first leg (18A) with the second leg (18B).

6. Device according to any of Claims 1 to 5,
characterized in that
the device possesses a hollow handle portion (14) having an anterior and a posterior end, said ends being located opposite each other, while the proximal end of the extended sleeve (16) is secured to the anterior end of the handle portion (14) and in that the lower jaw (42) is secured to the sleeve (16) at the distal end, an extended rod (25) being movably supported inside the sleeve (16), the rod (25) being secured at the distal end to the upper jaw (44).

7. Device according to any of Claims 1 to 6,
characterized in that
the handle portion (14) of the seizing means (10) possesses a gripping handle and a rotatably connected, spring-loaded triggering means (24).

8. Device according to Claim 7,
characterized in that
an elongated portion (23) of the triggering means (24) extends into the hollow handle portion (14) of the seizing means (10) and interacts with the rod (25) so as to move the rod (25) forwardly into the sleeve (16) when the triggering means (24) is pulled or moved.

9. Device according to Claim 8,
characterized in that
the device possesses a spring (26) which is located on the rod (25) between the elongated portion (23) of the triggering means (24) and the sleeve (16), in that, when the triggering means (24) is pulled or moved, the elongated portion (23) rotates in the forward direction within the handle portion (24) against the spring pressure while, in the process, it moves the rod (25) forwardly so that the upper jaw (44) moved in the direction towards the lower jaw (42) of the sleeve (16) so as to seize the tissue and in that, when the triggering means (24) is released, the elongated portion (23) and the rod (25) are pressed back and the triggering means (24) and the movable upper jaw (44) are urged into a normal open position.

## Revendications

1. Dispositif de refixation arthroscopique de tissus déchirés, en particulier de gaines de rotateurs déchirées, à l'os à un endroit devant être opéré chirurgiquement avec un ancrage de suture (30) pourvu d'un fil (8), un préhenseur de tissus (10) sur une gaine (16) munie d'une poignée (14) et un dispositif prévu sur le préhenseur de tissus pour retirer le fil,
caractérisé en ce
que la gaine (16) porte, à son extrémité proximale, la poignée (14) et à son extrémité distale, pouvant être introduite dans le patient à l'endroit devant être opéré, des mâchoires pivotantes (12) pour saisir le fil déchiré, que le dispositif pour retirer l'ancrage de suture (30) pourvu du fil (8) et le fil (8) qui se trouve sur l'ancrage de suture (30) pourvu du fil de l'endroit de l'opération, lorsque l'on enlève l'extrémité distale de la gaine (16) de l'endroit de l'opération est placé sur le préhenseur de tissus (10), l'ancrage de suture (30) pourvu du fil (8) avec une seule forure étant introduit dans l'os lors de la préhension simultanée du tissu au moyen du préhenseur de tissu (10), que deux mâchoires pivotantes (12) sont prévues à l'extrémité distale du préhenseur de tissus (10) pour saisir le tissu déchiré, les mâchoires (12) étant formées par une mâchoire supérieure (44) et une mâchoire inférieure (42), parmi lesquelles la mâchoire supérieure (44) est pourvue d'une ouverture (48) et la mâchoire inférieure (42) d'une rainure en forme d'U (46), l'ouverture (48) et la rainure (46) étant alignées l'une sur l'autre, lorsque les mâchoires (42, 44) sont fermées, et l'ancrage de suture (30) pourvu du fil (8) étant introduit dans l'os par l'ouverture (48) et la rainure (46) des mâchoires (42, 44) alignées l'une sur l'autre avec le tissu déchiré saisi.

2. Dispositif selon la revendication 1,
caractérisé en ce
que l'ancrage de suture (30) pourvu du fil (8) est introduit dans l'os par l'ouverture (48) et la rainure (46) des mâchoires (42, 44) alignées l'une sur l'autre en utilisant une canule (20) prévue sur le dispositif pour guider l'ancrage de suture (30) avec le tissu déchiré saisi, la canule (20) étant fixée à l'extrémité libre d'un pied d'appui (18) extensible relié à l'extrémité proximale de la gaine (16).

3. Dispositif selon la revendication 2,
caractérisé en ce
que le pied d'appui extensible (18) sert à loger un foret si bien que l'ancrage de suture (30) pourvu du fil (8) peut être automatiquement aligné sur l'ouverture (48) et la rainure (46) des mâchoires (42, 44) du moyen de préhension à l'endroit devant être opéré.

4. Dispositif selon l'une des revendications 2 et 3,
caractérisé en ce
que le pied d'appui extensible (18) présente une extrémité inférieure (17) et une extrémité supérieure (19), l'extrémité inférieure (17) étant creuse pour loger la gaine (16) du préhenseur (10), l'extrémité supérieure (19) étant pourvue d'une entrée de canule (29) pour loger une canule (20) par laquelle l'ancrage de suture (30) pourvu du fil (8) est poussé en direction de l'endroit à opérer.

5. Dispositif selon l'une des revendications 2 à 4,
caractérisé en ce
que le pied d'appui extensible (18) présente un premier montant (18A) qui s'étend à partir de l'extrémité inférieure (17), un second montant (18B) qui s'étend à partir de l'extrémité supérieure (19) et un montant de jonction (18C) qui relie le premier montant (18A) au second montant (18B).

6. Dispositif selon l'une des revendications 1 à 5,
caractérisé en ce
que le dispositif présente une pièce de poignée creuse (14) qui présente une extrémité antérieure et une extrémité postérieure qui sont situées l'une en face de l'autre, l'extrémité proximale de la gaine prolongée (16) étant fixée à l'extrémité antérieure de la pièce de poignée (14), et que la mâchoire inférieure (12) est fixée à la gaine (16) à l'extrémité distale, une tige prolongée (25) étant positionnée mobile à l'intérieur de la gaine (16), la tige (25) étant fixée à l'extrémité distale sur la mâchoire supérieure (44).

7. Dispositif selon l'une des revendications 1 à 6,
caractérisé en ce
que la pièce de poignée (14) du préhenseur (10) présente une poignée et un déclencheur (24) chargé par ressort, relié en rotation.

8. Dispositif selon la revendication 7,
caractérisé en ce
qu'un prolongement (23) du déclencheur (24) s'étend dans la pièce de poignée creuse (14) du préhenseur (10) et agit solidairement avec la tige (25) pour déplacer la tige (25) vers l'avant dans la douille (16), lorsque le déclencheur (24) est retiré ou déplacé.

9. Dispositif selon la revendication 8,
caractérisé en ce
que le dispositif présente un ressort (26) qui se trouve sur la tige (25) entre le prolongement (23) du déclencheur (24) et de la gaine (15), que, lorsque le déclencheur (24) est tiré ou déplacé, le prolongement (23) se tourne à l'intérieur de la pièce de poignée (24) à l'encontre de la pression du ressort vers l'avant, en déplacant la tige (25) vers l'avant si bien que la mâchoire supérieure (44) se déplace en direction de la mâchoire inférieure (42) de la gaine (16) pour saisir le tissu et que, lorsque le déclencheur (24) est relâché, le prolongement (23) et la tige (25) sont repoussés et le déclencheur (24) et la mâchoire supérieure mobile (44) sont poussés dans une position ouverte normale.
